# EUROPEAN PATENT APPLICATION

(11) **EP 4 671 382 A1**
(43) Date of publication of application: **31.12.2025**
(21) Application number: 24760611.4
(22) Date of filing: 23.02.2024
(51) Int. Cl.: C12Q 1/6881, C12N 5/0783

(54) **METHOD FOR SELECTING NK CELL DONOR SOURCE**

(30) Priority: 23.02.2023 KR 20230024481
(71) Applicant: Encell Co., Ltd., Gangnam-gu, Seoul 06072 (KR)
(72) Inventor: JEON, Hong Bae, Seoul 06072 (KR); PARK, Sang Rae, Seoul 06072 (KR); IM, Ji Hye, Seoul 06072 (KR)
(74) Representative: Mewburn Ellis LLP
(86) International application number: PCT/KR2024/002372
(87) International publication number: WO 2024/177422

(57) **Abstract**

The present invention relates to a method of selecting a donor source for NK cells with enhanced proliferative capacity, wherein specific single nucleotide polymorphisms (SNPs) of the CISH gene are identified. According to the present invention, in the context of cell therapy comprising NK cells, the method addresses the issue of varying efficacy of NK cells depending on the donor source, thereby enabling the production of NK cells with superior proliferative capacity and consistent quality.

## Description

### [Technical Field]

The present invention relates to a method of selecting a donor source capable of obtaining NK cells with enhanced cell proliferation ability, and more specifically, to a method of selecting a donor source for NK cells with enhanced proliferation capacity by identifying a specific single nucleotide polymorphism of the CISH gene.

### [Background Art]

Natural killer cells (hereinafter, simply referred to as "NK cells"), among the cells constituting the immune system, are known to be immune cells capable of killing virus-infected cells and cancer cells through a comprehensive balance of activating and inhibitory receptor signaling, even without being sensitized to specific antigens. This killing ability of NK cells is used alone or in combination with other therapies to treat various hematologic malignancies and solid tumors. Drugs such as cytokines may be injected into the body of patients to activate NK cells and achieve anticancer effects, and other methods involve activating and amplifying NK cells or genetically engineered NK cells *ex vivo,* and then injecting the cells into the patient.

To effectively utilize NK cells in anticancer immunotherapy, securing a large number of NK cells is necessary. However, NK cells account for 10 to 15% of lymphocytes in the blood, and cancer patients often have reduced numbers of NK cells, and deteriorated differentiation and functions thereof, making it difficult to secure sufficient numbers. Therefore, there is an urgent need to secure large quantities of NK cells through proliferation or differentiation thereof.

NK cells are typically derived from hematopoietic stem cells (HSCs) in the bone marrow and are known to be distributed throughout various tissues and blood. Representative sources include the spleen, liver, bone marrow, peripheral blood, and umbilical cord blood. Methods including isolating hematopoietic stem cells (HSCs) *in vitro* from umbilical cord blood and culturing the same with appropriate cytokines to differentiate the same into NK cells have been reported.

Conventional methods of proliferating NK cells involve the use of high concentrations of various expensive cytokines or the use thereof in combination with peripheral blood mononuclear cells (PBM) or cancer cell lines. These methods were not only expensive but also resulted in low amplification rates. However, recently, methods utilizing genetically engineered cancer cell lines (Imai C et al., Blood. 2005;106:376-83; Denman CJ et al., PLoS One . 2012;7:e30264; Thangaraj JL et al., Cancer Immunol Immunother. 2022;71:613-625) have been introduced to overcome many of these limitations. However, in order to improve the NK cell proliferation rate, various culture conditions such as cytokine composition, nutrient cell characteristics, medium composition, and the ratio of nutrient cells and mononuclear cells (peripheral blood, umbilical cord blood) must be optimized to achieve the best amplification. In addition, a number of studies have shown proliferation of other lymphocytes such as T cells in addition to NK cells, thus making NK cell-based immune cell therapy unsuitable. Therefore, a method of selecting only NK cells with high proliferation rate and high cancer cell killing ability is required.

Accordingly, in the present invention, as a result of extensive efforts to develop a method of selecting a subject sample capable of isolating NK cells with enhanced cell proliferation ability, it was found that NK cells isolated from a source in which the allele type at rs414171 among single nucleotide polymorphisms (SNPs) of the CISH (cytokine-inducible SH2-containing protein) gene is TT have a significantly higher proliferation rate than those of other genotypes. Based on this finding, the present invention has been completed.

### [Disclosure]

It is one object of the present invention to provide a method of selecting a donor source for isolating NK cells with high cell proliferation ability.

In accordance with one aspect of the present invention, the above and other objects can be accomplished by the provision of a method of selecting a donor source for NK cells including:
(a) identifying a genotype at rs414171, which is a single nucleotide polymorphism site, of a CISH gene in the genome of a donor-derived sample; and
(b) selecting a donor source having an AT genotype or a TT genotype at rs414171 as the donor source for NK cells.

In accordance with another aspect of the present invention, provided is a method of culturing NK cells including:
(a) isolating NK cells from the donor source selected by the method; and
(b) co-culturing the NK cells with feeder cells to culture the NK cells.

In accordance with another aspect of the present invention, provided are NK cells cultured by the method.

### [Description of Drawings]

FIG. 1 shows the results of genotyping of CISH single nucleotide polymorphism (rs414171) using umbilical cord blood.
FIG. 2 shows the results of analysis of NK cell proliferation rate and purity according to CISH genotype.

### [Best Mode]

Unless defined otherwise, all technical and scientific terms used herein have the same meanings as those appreciated by those skilled in the field to which the present invention pertains. In general, the nomenclature used herein is well-known in the art and is ordinarily used.

In the present invention, in order to solve the problem of the difference in the efficacy of NK cells depending on the difference in the donor source, the genotype of the single nucleotide polymorphism (SNP) of the donor was identified, the proliferation ability of the NK cells isolated depending on the genotype was determined, and it was found that there was a difference in the proliferation ability of the isolated NK cells depending on the genotype of the rs414171 SNP derived from the CISH (cytokine-inducible SH2-containing protein) gene in umbilical cord blood.

In one aspect, the present invention is directed to a method of selecting a donor source for NK cells including:
(a) identifying a genotype at rs414171, which is a single nucleotide polymorphism site, of a CISH gene in the genome of a donor-derived sample; and
(b) selecting a donor source having an AT genotype or a TT genotype at rs414171 as the donor source for NK cells.

The CISH gene, which is a member of the SOCS (suppressor of cytokine signaling) protein family, acts as a negative regulator of IL-15-mediated signaling in NK cells. It has been reported that CISH-deficient NK cells exhibit enhanced proliferation, viability, and cytotoxicity (Huang Zhu, Cell Stem Cell, 6;27(2):224-237, 2020).

The rs414171 SNP, which is a single nucleotide polymorphism in the CISH gene, has been reported to be associated with the incidence of infectious diseases such as tuberculosis and sepsis, depending on the mutation (Alizada, Azad, University of Toronto (Canada) ProQuest Dissertations Publishing, 2017. 10634550).

In the present invention, the donor-derived sample may be peripheral blood or umbilical cord blood.

In the present invention, in step (b), a donor source having a TT genotype at rs414171 is selected as the donor source for NK cells.

In one embodiment of the present invention, the genotype of the CISH single nucleotide polymorphism (rs414171) was analyzed using tetra-primer amplification refractory mutation system-PCR (T-ARMS-PCR). However, any method may be used without limitation as long as it is generally capable of identifying SNP sequences.

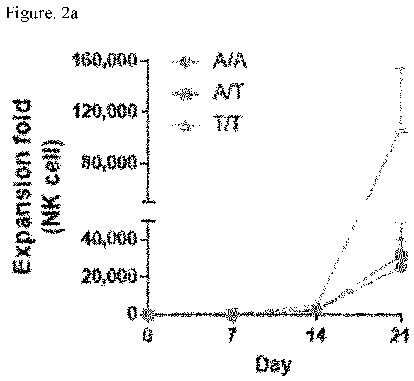

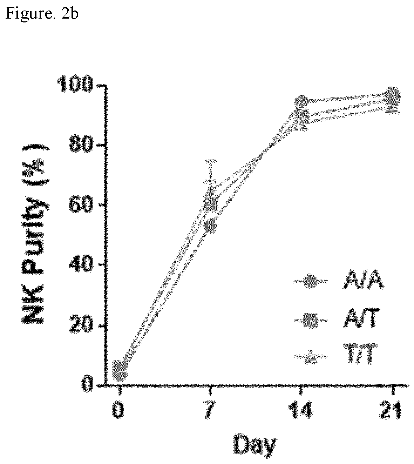

In one embodiment of the present invention, it was found that the proliferation rate of NK cells derived from a sample having a T/T allele genotype in the single nucleotide polymorphism rs414171 was significantly higher than that of a sample having an A/A or A/T allele genotype (FIG. 2).

As used herein, the term "natural killer cells" or "NK cells" refers to cytotoxic lymphocytes that constitute a major component of the innate immune system, and are defined as large granular lymphocytes (LGLs). The "natural killer cells" or "NK cells" include natural killer cells that undergo no further modification derived from any tissue source, and may include mature natural killer cells as well as natural killer precursor cells. The natural killer cells are activated in response to interferon or macrophage-derived cytokines, and natural killer cells contain two types of surface receptors that control the cytotoxic activity of the cells, labeled "activating receptors" and "inhibitory receptors". Natural killer cells may be produced from hematopoietic cells, such as hematopoietic stems or precursors, from any source, such as placental tissue, placental perfusate, umbilical cord blood, placental blood, peripheral blood, spleen, liver, and the like.

In one embodiment, the natural killer cell may be an activated natural killer cell. The activated natural killer cell may refer to a cell in which cytotoxicity or the inherent immunomodulatory capacity of a natural killer cell is activated compared to parent cells, such as hematopoietic cells or natural killer precursor cells.

In one embodiment, non-limiting examples of the NK cell include macrophages, B lymphocytes, T lymphocytes, mast cells, monocytes, dendritic cells, eosinophils, natural killer cells, basophils, and neutrophils. Accordingly, in certain embodiments, the NK cell may include any one selected from the group consisting of macrophages, B lymphocytes, T lymphocytes (CD8+ CTLs), mast cells, monocytes, dendritic cells, eosinophils, natural killer cells, basophils, and neutrophils. In certain embodiments, the NK cell may be a natural killer cell or a T lymphocyte. The mixed NK cells may include one or more selected from the group consisting of macrophages, B lymphocytes, T lymphocytes, mast cells, monocytes, dendritic cells, eosinophils, natural killer cells, basophils, and neutrophils.

In one embodiment, activated natural killer cells or a population enriched in activated natural killer cells may be evaluated by detecting at least one functionally relevant marker, such as CD16, CD57, CD69, CD94, CD161, CD158a, CD158b, NKp30, NKp44, NKp46, DNAM-1, 2B4, NKp46, CD94, KIR (e.g., KIR2DL1, KIR2DL2/3, KIR3DL1), and the NKG2 family of activating receptors (e.g., NKG2A, NKG2C, NKG2D).

In another aspect, the present invention is directed to a method of culturing NK cells including:
(a) isolating NK cells from the donor source selected by the method; and
(b) co-culturing the NK cells with feeder cells to culture the NK cells.

In the present invention, the feeder cells may be K562 cells that express membrane-bound IL-18 and membrane-bound IL-21 on the their surfaces.

In another aspect, the present invention is directed to NK cells cultured by the method described above.

As used herein, the term "stimulation of NK cells" may refer to increasing the activity, e.g., cytotoxic activity, of natural killer cells, *in vitro* or *in vivo,* or to the generation, increase, expansion, or proliferation of activated natural killer cells.

In one aspect of the present invention, in an embodiment, a cell line genetically engineered to express membrane-bound interleukin-18 (mbIL-18) and membrane-bound interleukin-21 (mbIL-21) is used as the feeder cells.

As used herein, the term "feeder cells" (also referred to as "support cells", "culture aid cells", or "food cells") may refer to cells that do not have the ability to divide and proliferate upon radiation exposure, but have metabolic activity and thus produce various metabolites, thereby facilitating the proliferation of target NK cells. Examples of feeder cells that may be used herein include genetically engineered animal cell lines, such as human chronic myelogenous leukemia cell lines (e.g., K562 cells), RPMI8866, EBV_LCL, 721221, and HFWT. In one embodiment of the present invention, K562 cells are used.

As used herein, the term "genetic engineering" or "genetically engineered" refers to an action of introducing at least one genetic modification into a cell, or to a cell produced thereby.

Specifically, the genetically engineered cell may include an exogenous gene encoding the aforementioned gene. The term "exogenous" refers to a property in which the referenced molecule or activity is introduced into a host cell. The molecule may be introduced into the host genetic material as an encoding nucleic acid, such as by insertion into the host chromosome, or as non-chromosomal genetic material, such as a plasmid. With respect to the expression of the encoding nucleic acid, the term "exogenous" refers to the introduction of the encoding nucleic acid into the organism in an expressible form. With respect to biosynthetic activity, the term "exogenous" refers to the activity introduced into the host parent cell. The source may be, for example, a homologous or heterologous coding nucleic acid that expresses the activity after being introduced into a host cell. Therefore, the term "endogenous" refers to the molecule or activity present in the host cell. Similarly, with respect to the expression of a coding nucleic acid, the term "endogenous" refers to the expression of the coding nucleic acid contained within the organism. The term "heterologous" refers to a molecule or activity from a source other than the mentioned species, and the term "homologous" refers to a molecule or activity from the host cell. Therefore, exogenous expression of a coding nucleic acid may use either or both heterologous and homologous coding nucleic acids.

Therefore, the genetically engineered feeder cells of the present invention may include nucleic acids encoding mbIL-18 and mbIL-21. More specifically, the cells may be transformed with a vector containing a nucleic acid encoding mbIL-18 and mbIL-21.

As used herein, the term "vector" refers to a means for expressing target proteins in appropriate host cells, and includes a genetic construct that contains a regulatory element operably linked to a gene insert to express the gene insert. In one embodiment, the vector may include an expression control element such as a promoter, operator, initiation codon, termination codon, polyadenylation signal, and/or enhancer, and the promoter of the vector may be constitutive or inducible. Furthermore, the vector may be an expression vector capable of stably expressing the fusion protein in a host cell. The expression vector may be any common vector used in the art to express foreign proteins in plants, animals, or microorganisms. The recombinant vector may be constructed using various methods known in the art. For example, the vector may include a selectable marker for selecting host cells containing the vector, and when the vector is replicable, it may include an origin of replication. Furthermore, the vector may be self-replicated or integrated into host DNA, and may be selected from the group consisting of plasmids, lentiviruses, adenoviruses, adeno-associated viruses, retroviruses, herpes simplex viruses, and vaccinia viruses.

In addition, in the vector, the polynucleotide sequence encoding the aforementioned fusion protein may be operably linked to a promoter. As used herein, the term "operably linked" refers to a functional linkage between a nucleic acid expression control sequence (e.g., a promoter, a signal sequence, or an array of transcription factor binding sites) and another nucleic acid sequence, and enables the control sequence to regulate the transcription and/or translation of the other nucleic acid sequence.

As used herein, the term "membrane-bound interleukin" refers to an interleukin bound to the cell membrane and may be different from interleukin secreted extracellularly.

In one embodiment, the co-culture may be performed in the presence of a cytokine.

In one embodiment, the cytokine is an interleukin or a mutant thereof. Many interleukins are synthesized by helper CD4 T lymphocytes, as well as monocytes, macrophages, and endothelial cells. Interleukins may promote the development and differentiation of T and B lymphocytes and hematopoietic cells. Non-limiting examples of interleukins include IL1, IL2, IL3, IL4, IL5, IL6, IL7, IL8 (CXCL8), IL9, IL10, IL11, IL12, IL13, IL14, IL15, IL16, IL17, IL18, IL19, IL20, IL21, IL22, IL23, IL24, IL25, IL26, IL27, IL28, IL29, IL30, IL31, IL32, IL33, IL35, or IL36. Therefore, in some embodiments, the cytokine is an interleukin or a mutant thereof, including (but not limited to), wild-type and mutants of IL1, IL2, IL3, IL4, IL5, IL6, IL7, IL8 (CXCL8), IL9, IL10, IL11,IL12, IL13, IL14, IL15, IL16, IL17, IL18, IL19, IL20, IL21, IL22, IL23, IL24, IL25, IL26, IL27, IL28,IL29, IL30, IL31, IL32, IL33, IL35, or IL36.

In another embodiment, the cytokine added during the co-culture may be IL-2. More specifically, the IL-2 may be used at a concentration of 1 to 200 U/mL, preferably 1 to 100 U/mL, more preferably 1 to 50 U/mL, and most preferably 1 to 20 U/mL.

In another embodiment, the cytokine added during the restimulation may be IL-2. More specifically, the IL-2 may be used at a concentration of 10 to 500 U/mL, preferably 10 to 300 U/mL, and more preferably 10 to 250 U/mL.

### [Example]

Hereinafter, the present invention will be described in more detail with reference to the following examples. However, it will be obvious to those skilled in the art that the following examples are provided only for illustration of the present invention, and should not be construed as limiting the scope of the present invention.

### Example 1. Genotyping of CISH single-nucleotide polymorphism (rs414171)

About 200 µL of cord blood (Samsung Medical Center, Seoul) was collected from a healthy donor and genomic DNA (gDNA) was extracted using the QIAamp DNA mini kit (QIAGEN, Hilden, Germany). The extracted gDNA was diluted in nuclease-free distilled water to a concentration of 10 ng/µL, which was used as template DNA.

The genotype of the CISH single-nucleotide polymorphism (rs414171) was analyzed using tetra-primer amplification refractory mutation system-PCR (T-ARMS-PCR).

Two external primers were used as positive controls for the CISH gene, and two internal primers were used to detect the A or T allele of the 292^{nd} position genotype (rs414171) of the CISH. The specific nucleotide sequences are as follows.

**[Table 1]**

| Internal primers for detecting rs414171 alleles | | |
|---|---|---|
| rs414171 | Strand | Primer sequence |
| External primers | Forward (SEQ ID NO: 1) | ACGCCGACAGACCTCCTTGGAGGAG |
| | Reverse (SEQ ID NO: 2) | GAAGCAGCGTCTTCCTAGAACCGCGG |
| Internal primers | Forward (T allele) (SEQ ID NO: 3) | TGCTATTGGCCCTCCCCGACCACT |
| | Reverse (A allele) (SEQ ID NO: 4) | CGCGACGCTGAAGGTGGAGCTGT |

Genomic DNA (50 ng), four primers (0.5 µM each), and nuclease-free distilled water were mixed to adjust a total volume of 20 pL, the resulting mixture was mixed with AccuPower PCR PreMix (Bioneer, Korea) and amplified by PCR using a VeritiPro Thermal Cycler (Applied Biosystems, CA, USA). The PCR reaction was performed by pre-denaturation at 95°C for 5 min (1 cycle), 30 cycles, each cycle including denaturation at 95°C for 30 seconds, annealing at 68°C for 30 seconds, and extension at 72°C for 30 seconds, and final extension at 72°C for 5 minutes. The PCR product was diluted at 1/10 in nuclease-free distilled water, loaded onto 2% E-Gel^{™}EX Agarose Gels (Invitrogen, CA, USA) at a volume of 20 µL per sample, and subjected to electrophoresis and gel imaging using an E-Gel^{™}Power Snap Electrophoresis System (Invitrogen, CA, USA) to obtain the results.

**[Table 2]**

| PCR conditions | | | |
|---|---|---|---|
| Step | Temperature | Time | Cycle |
| Pre-denaturation | 95°C | 5 min | 1 cycle |
| Denaturation | 95°C | 30 sec | 30 cycles |
| Annealing | 68°C | 30 sec | |
| Extension | 72°C | 30 sec | |
| Final extension | 72°C | 5 min | 1 cycle |

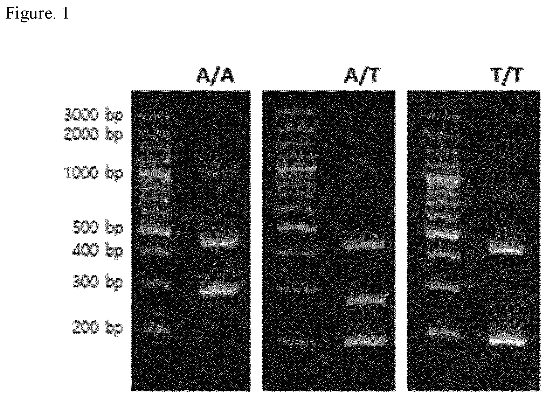

In all samples, a 452-bp band of the CISH control gene is detected. When the 292^{nd} position genotype (rs414171) of the CISH gene is A, a 290-bp band is further detected. When the genotype is T, a 208-bp band is further detected. Depending on the homozygous or heterozygous genotype, three bands can be detected: A/A, A/T, and T/T (FIG. 1).

Example 2. Selection of donors with T/T genotype at CISH single nucleotide polymorphism (rs414171) and isolation of blood-derived mononuclear cells (MNC)

### 2-1: Screening of T/T homozygotes at rs414171

Using the method described in Example 1, samples having a T/T homozygote as the 292^{nd} position genotype (rs414171) of the CISH were screened from cord blood collected from healthy donors (Samsung Medical Center). These samples were then supplemented with 2% (v/v) fetal bovine serum (FBS, ThermoFisher, USA) at a ratio of 1:1.

### 2-2: Mononuclear cell isolation

Mononuclear cells were isolated from cord blood using Lymphoprep (Serumwerk, Bernburg AG, Oslo, Norway) density gradient centrifugation, then suspended in NK MACS medium (MiltenyiBiotec, Bergisch Gladbach, FRG) containing 20 U/mL IL-2 (Peprotech, Rocky Hill, NJ), 1% (v/v) NK MACS Supplements, and 10% (v/v) human AB serum (Sigma, USA) and the number of cells was measured using a LUNA-FX7 automated cell counter (Aligned Genetics, Korea).

The proportion of NK cells in mononuclear cells was determined to determine the number of feeder cells seeded at a co-culture ratio (1:5) of natural killer (NK) cells and genetically engineered feeder cells. Specifically, 2.0 x 10⁵ mononuclear cells were harvested, washed with DPBS, and stained with Fixable Viability Stain 780 (BD Biosciences, San Jose, CA) for 15 minutes. The cells were then washed with DPBS buffer containing 2% (v/v) FBS and further stained with anti-human CD3-FITC and anti-human CD56-APC (BD Biosciences, San Jose, CA) for 30 minutes at 4°C. The cells were then washed again with DPBS containing 2% (v/v) FBS. Data were acquired using CytoFLEX (Beckman Coulter, Brea, CA) and analyzed with FlowJo software (Tree Star, Ashland, OR).

### 2-3: Preparation of genetically engineered feeder cells

K562 cells expressing membrane-bound IL-18 and membrane-bound IL-21 on the their surfaces were produced using the method described in Korean Patent Publication No. 10-2021-0152934. The genetically engineered K562-mbIL-18/21 cell line was cultured in a T-flask with an appropriate size containing RPMI1640 (Gibco, USA) medium containing 10% (v/v) FBS in a 37°C, 5% CO₂ incubator. The cells were recovered from the culture flask, centrifuged at 400 g for 3 minutes, suspended in RPMI1640 medium containing 10% (v/v) FBS, and inactivated by irradiation with 100 Gy in a Gammacell 3000 Elan gamma irradiator. The cells were prepared by centrifugation again at 400 g for 3 minutes and by suspending in NK MACS medium containing 20 U/mL IL-2, 1% (v/v) NK MACS Supplements, and 10% (v/v) human AB serum.

### [Example 3: Natural killer cell culture using mononuclear cells with T/T genotype of CISH single nucleotide polymorphism (rs414171)

On day 0 of culture, the cord blood-derived mononuclear cells were seeded at a density of 1.25 x 10⁵ cells/cm² into G-Rex24 culture vessels (Wilson Wolf, Saint Paul, MN). Genetically engineered feeder cells were seeded at a ratio of 1:5 relative to the number of natural killer cells in the cord blood-derived mononuclear cells. NK MACS medium containing 20 U/mL IL-2, 1% (v/v) NK MACS supplements, and 10% (v/v) human AB serum was added to a total volume of 8 mL, and the result was cultured in a 37°C, 5% CO₂ incubator for 7 days.

During the 7-day culture period, about 20 µL of culture supernatant was collected every 3 to 5 days. Glucose concentration was measured using a CareSens N Voice blood glucose meter (i-Sens, Korea). When the glucose concentration was below 50 to 100 mg/dL, 4 mL of culture supernatant was carefully removed and replaced with 4 mL of NK MACS medium containing 20 U/mL IL-2, 1 v/v% NK MACS supplement, and 10% (v/v) human AB serum to replace the half of the total medium volume.

On day 7 of culture, the culture supernatant from the G-Rex24 culture vessel was carefully removed to restimulate genetically engineered feeder cells. The number of cells was then measured using a LUNA-FX7 automated cell counter. 2.0x10⁵ cultured cells were harvested in accordance with the instructions in Example 2 to determine the purity of natural killer cells. Based on the total cell number, the cell density was maintained at 1.25 x 10⁵ to 4.0 x 10⁷ cells/cm², and the genetically engineered feeder cells prepared in accordance with the instructions in Example 2 were seeded thereon at a ratio of 1:5 of the genetically engineered feeder cells to the number of cultured natural killer cells for restimulation. Then, the cells were cultured in a total volume of 8 mL of NK MACS medium containing 200 U/mL IL-2, 10 ng/mL IL-15, 1% (v/v) NK MACS supplements, and 10% (v/v) human AB serum.

During the 21-day culture period, the glucose concentration was measured every 3 to 5 days, and the culture medium was replaced by half at the appropriate time. In addition, the cell count was measured every 5 to 7 days to determine the subculture period. Based on the total cell number, the cells were diluted at a cell density of 1.25 x 10⁵ to 4.0 x 10⁷ cells/cm² in NK MACS medium containing 200 U/mL IL-2, 10 ng/mL IL-15, 1% (v/v) NK MACS supplements, and 10% (v/v) human AB serum.

### Example 4: Determination of purity and expansion rate of natural killer cells cultured from mononuclear cells with T/T genotype at CISH single nucleotide polymorphism (rs414171)

In the cultures according to Example 3, the total cell count was measured on days 7 and 14 using a LUNA-FX7 automated cell counter. The purity of the natural killer cells was analyzed using Fixable Viability Stain 780, anti-human CD3-FITC, and anti-human CD56-APC, as described in Example 2. The total number of natural killer cells calculated at 7-day intervals was compared with the number of natural killer cells on day 0 of the culture to evaluate the proliferation rate.

As shown in FIG. 2, the result showed that the proliferation rate of NK cells derived from specimens with the T/T genotype at the single nucleotide polymorphism rs414171 was significantly higher than that of the A/A or A/T alleles.

### [Industrial Applicability]

In the present invention, NK cells with excellent proliferation ability and consistency can be produced by solving the problem of the efficacy of NK cells varying depending on the difference in the donor source in cell therapeutics containing NK cells.

Although specific configurations of the present invention have been described in detail, those skilled in the art will appreciate that this description is provided to set forth preferred embodiments for illustrative purposes and should not be construed as limiting the scope of the present invention. Therefore, the substantial scope of the present invention is defined by the accompanying claims and equivalents thereto.

### [Sequence Listing Free Text]

An electronic file is attached.

## Claims

1. A method of selecting a donor source for NK cells comprising:
(a) identifying a genotype at rs414171, which is a single nucleotide polymorphism site, of a CISH gene in the genome of a donor-derived sample; and
(b) selecting a donor source having an AT genotype or a TT genotype at rs414171 as the donor source for NK cells.

2. The method according to claim 1, wherein the donor-derived sample is peripheral blood or umbilical cord blood.

3. The method according to claim 1, wherein in step (b), a donor source having a TT genotype at the rs414171 is selected as the donor source for NK cells.

4. A method of culturing NK cells comprising:
(a) isolating NK cells from the donor source selected by the method according to any one of claims 1 to 3; and
(b) co-culturing the NK cells with feeder cells to culture the NK cells.

5. The method according to claim 4, wherein the feeder cells are K562 cells expressing membrane-bound IL-18 and membrane-bound IL-21 on the their surfaces.

6. NK cells cultured by the method according to claim 4.
